(19) Europäisches Patentamt
European Patent Office
Office européen des brevets

(11) EP 4 810 031 A1

(12) **EUROPEAN PATENT APPLICATION**
published in accordance with Art. 153(4) EPC

(43) Date of publication:
**23.09.2026 Bulletin 2026/39**

(21) Application number: **24891879.9**

(22) Date of filing: **14.11.2024**

(51) International Patent Classification (IPC):
***A61K 9/70** (2006.01)* ***A61K 31/192** (2006.01)*
***A61P 29/00** (2006.01)*

(52) Cooperative Patent Classification (CPC):
**A61K 9/70; A61K 31/192; A61P 29/00**

(86) International application number:
**PCT/KR2024/096568**

(87) International publication number:
**WO 2025/105933 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.11.2023 KR 20230158673**

(71) Applicant: **Handok Inc.**
**Seoul 06235 (KR)**

(72) Inventors:
- **JI, Eun Kyoung**
  **Seoul 04702 (KR)**
- **JUN, Sang Hui**
  **Anyang-si, Gyeonggi-do 13994 (KR)**
- **KIM, Kyung Mi**
  **Seoul 02567 (KR)**
- **LEE, Young Eun**
  **Bucheon-si, Gyeonggi-do 14479 (KR)**
- **PARK, Hyun Jung**
  **Seoul 07605 (KR)**

(74) Representative: **Engelhard, Markus**
**Boehmert & Boehmert**
**Anwaltspartnerschaft mbB**
**Pettenkoferstrasse 22**
**80336 München (DE)**

# (54) TRANSDERMAL PREPARATION CONTAINING FLURBIPROFEN WITH IMPROVED STABILITY AND PERMEABILITY

(57) The present invention provides a transdermal preparation having a multi-layered structure comprising a hydrophobic adhesive layer and a drug adhesive layer, wherein the structure of each layer and the ratio of adhesives contained therein are optimized. The transdermal preparation of the present invention can increase the skin permeability of a drug without using a skin permeation enhancer, and exhibits excellent adhesion and product stability without causing skin irritation, and thus can be advantageously used as a transdermal delivery system of a drug in the pharmaceutical and medical fields.

【Figure 1】

1) an elastic backing layer
2) a hydrophobic adhesive layer
3) a drug adhesive layer
4) a release layer

EP 4 810 031 A1

## Description

## TECHNICAL FIELD

This application claims priority to Korean Patent Application No. 10-2023-0158673, filed on November 15, 2023, the entire disclosure in the specification and drawings of which is incorporated herein by reference.

The present invention relates to a transdermal delivery preparation with improved stability and permeability, and more specifically, to a transdermal delivery preparation with improved stability and permeability comprising flurbiprofen.

## BACKGROUND ART

Transdermal drug delivery systems are designed to target drugs that are absorbed into the skin or, mainly, the subcutaneous layer, and a variety of drugs, including non-steroidal anti-inflammatory drugs, which are representative drugs developed as transdermal drug delivery systems, are being developed as transdermal drug delivery systems.

Nonsteroidal anti-inflammatory drugs relieve inflammation or pain, and various drugs such as ketoprofen, flurbiprofen, diclofenac, piroxicam, indomethacin, felbinac, ibuprofen, and loxoprofen have been developed and sold as transdermal preparations.

Transdermal delivery preparations have the advantage of being simple to administer and having fewer side effects of gastrointestinal disorders compared to oral delivery preparations.

However, since the absorption and action of transdermal delivery preparations can vary greatly between individuals and can differ depending on the characteristics of the skin, individual administration methods and dosage adjustments may be necessary, especially for children, the elderly, and patients with skin lesions. In addition, if transdermal delivery preparations are used for a long time, side effects such as skin irritation, itching, allergic reactions, redness, and dermatitis may occur due to skin penetration enhancers contained in the preparation. In addition, skin irritation and allergic reactions to such skin penetration enhancers can vary greatly from individual to individual, so even if no side effects were observed in skin irritation tests during product development, unexpectedly severe irritation or rashes may occur depending on the user during actual use, which is inconvenient.

There is a need to develop a transdermal delivery formulation that has excellent drug penetration and improved product stability without using skin penetration enhancers that may cause skin irritation.

Korean Patent No. 10-00663163 disclose a transdermal delivery preparation comprising a non-steroidal anti-inflammatory analgesic, which is a transdermal drug delivery system having a layered structure characterized by comprising a elastic backing layer, a hydrophobic adhesive layer, and a drug adhesive layer. However, there has been no research on a transdermal flurbiprofen formulation that improves both product stability and skin permeability, as in the present invention.

## DISCLOSURE

### Technical Problem

The problem to be addressed by the present invention is to provide a transdermal delivery preparation having excellent skin permeability, improved stability, and no skin irritation without using a skin penetration enhancer.

The problem to be addressed by the present invention is not limited to the problems mentioned above, and other technical problems not mentioned can be clearly understood by those skilled in the art from the description below.

### Technical Solution

To address the problem above, according to one aspect of the present invention, there is provided a transdermal delivery preparation which is a multilayered transdermal delivery formulation, said preparation comprising: an elastic backing layer (1); a hydrophobic adhesive layer (2), the first side of the hydrophobic adhesive layer is in contact with one side of the elastic backing layer (1) and the hydrophobic adhesive layer (2) contains a rubber-based adhesive; a drug adhesive layer (3), the first side of the drug adhesive layer (3) is in contact with the second side of the hydrophobic adhesive layer (2), and the drug adhesive layer (3) contains an acrylic-based adhesive and a drug ; and a release layer (4), one side of the release layer (4) is in contact with the second side of the drug adhesive layer (3), wherein the acrylic-based adhesive is a mixture of an acrylic-based adhesive with a functional group and an acrylic-based adhesive without a functional group in a ratio of 40:60 to 90:10 % by solid weight, wherein the acrylic-based adhesive with a functional group has functional groups of carboxyl group (-COOH) and hydroxyl group (-OH), wherein the drug contained in the drug adhesive layer (3) is flurbiprofen.

## Advantageous Effects

According to the present invention, in a multilayered transdermal drug delivery formulation comprising a hydrophobic adhesive layer and a drug adhesive layer, it has been found that by optimizing the structure of each layer and the ratio of the adhesive contained therein, the skin permeability of the drug can be increased without using a skin penetration enhancer, and side effects that vary from individual to individual can be avoided by not using a skin penetration enhancer. In addition, it has been confirmed that the transdermal drug delivery formulation of the present invention exhibits excellent adhesiveness, while also having excellent product stability (content stability and related substance generation) and being free of skin irritation. Therefore, the transdermal drug delivery formulation of the present invention can be usefully used as a transdermal drug delivery system in the fields of pharmacy and medicine.

The effects of the present invention are not limited to the effects described above, and should be understood to include all effects that can be inferred from the composition of the invention described in the description or claims of the present invention.

## DESCRIPTION OF DRAWINGS

**Figure 1** is a schematic diagram showing the matrix structure of the transdermal delivery preparation of the present invention.

**Figure 2** is a graph showing the skin penetration amount ($\mu g/cm^2$) of flurbiprofen of products to which Comparative Examples 1 to 3 and Examples 1 to 3 were applied.

**Figure 3** is a graph showing the skin penetration ratio (%) of flurbiprofen of products to which Comparative Examples 1 to 3 and Examples 1 to 3 were applied.

**Figure 4** is a graph showing the skin penetration amount ($\mu g/cm^2$) of flurbiprofen for 24 hours in Example 2 and third-party products.

**Figure 5** is a graph showing the skin penetration ratio (%) of flurbiprofen for 24 hours in Example 2 and third-party products.

**Figure 6** is a graph showing skin penetration amount ($\mu g/cm^2$) of flurbiprofen of products to which examples 2 to 5 were applied.

**Figure 7** is a graph showing the skin penetration ratio (%) of flurbiprofen of products to which Examples 2 to 5 were applied.

**Figure 8** is a graph showing skin penetration amount ($\mu g/cm^2$) of flurbiprofen of Examples 4 to 5 and third-party products.

**Figure 9** is a graph showing the skin penetration ratio (%) of flurbiprofen of Examples 4 to 5 and third-party products.

**Figure 10** is a graph showing changes in the flurbiprofen content of Comparative Examples 1 to 3 and Examples 1 to 3 under stress storage conditions.

**Figure 11** is a graph showing changes in the total amount of related substances generated from flurbiprofen in Comparative Examples 1 and 2 and Examples 1 and 2 under stress storage conditions.

**Figure 12** is a design for applying test materials to rabbit abdomen for skin irritation testing.

## BEST MODE

The present invention is provides a transdermal delivery preparation which is a multilayered transdermal delivery formulation, said preparation comprising: an elastic backing layer (1); a hydrophobic adhesive layer (2), the first side of the hydrophobic adhesive layer is in contact with one side of the elastic backing layer (1) and the hydrophobic adhesive layer (2) contains a rubber-based adhesive; a drug adhesive layer (3), the first side of the drug adhesive layer (3) is in contact with the second side of the hydrophobic adhesive layer (2), and the drug adhesive layer (3) contains an acrylic-based adhesive and a drug ; and a release layer (4), one side of the release layer (4) is in contact with the second side of the drug adhesive layer (3), wherein the acrylic-based adhesive is a mixture of an acrylic-based adhesive with a functional group and an acrylic-based adhesive without a functional group in a ratio of 40:60 to 90:10 % by solid weight, wherein the acrylic-based adhesive with a functional group has functional groups of carboxyl group (-COOH) and hydroxyl group (-OH), wherein the drug contained in the drug adhesive layer (3) is flurbiprofen.

The term "about" as used herein means a range of +/- 10% of the numerical value to which it refers, and includes all numerical values in a range equal to or similar to the numerical value following the term "about".

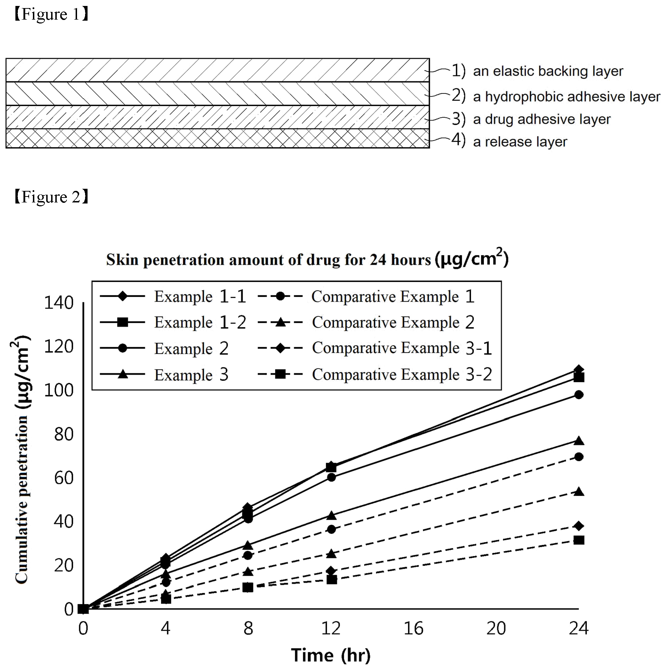

In the present invention, the drug adhesive layer (acrylic-based adhesive layer, acrylic layer) (3) has a structure in which one side is in contact with a hydrophobic adhesive layer (2) and the other side is in contact with a release layer (4) (see Fig. 1), and which includes an acrylic-based adhesive and a drug.

The drug contained in the drug adhesive layer (3) may be contained in an amount of about 5 to 40 weight % based on the total weight of the drug adhesive layer (3). Preferably, the drug may be contained in an amount of about 10 to 30 weight %, about 12 to 25 weight %, about 13 to 21 weight %, about 14 to 20 weight %, about 14 to 16 weight %, or about 18 to 20 weight % based on the total weight of the drug adhesive layer (3).

The drug adhesive layer (3) containing flurbiprofen shows a slight change in adhesiveness after containing the main ingredient flurbiprofen compared to before containing the main ingredient. The component, flurbiprofen, makes the physical properties of the adhesive layer more flexible, thereby imparting soft physical properties. The drug adhesive layer (3) shows the best adhesiveness when the content of flurbiprofen, which is an API, is in the range of about 13 to 21 weight % based on the total weight of the drug adhesive layer.

In one embodiment, the drug adhesive layer (3) can be prepared to have a thickness of about 10 to 40 μm, preferably about 19 to 30 μm.

The thickness can be estimated by dividing the weight of the drug adhesive layer by the area of the plaster (70 $cm^2$) (assuming a density of 1, g = $cm^3$). A more accurate thickness can be determined by applying the density of the matrix. For example, if the weight of the drug adhesive layer is 138.0 mg/plaster, the thickness can be estimated to be approximately 19.7 μm (assuming a density of 1).

$$(138.0 \text{ x } 10^{-3} \text{ cm}^3) / (70 \text{ cm}^2) = 1.9714285 \text{ x} 10^{-3} \text{ x } 10^{-2} \text{ m} \fallingdotseq 19.7 \text{ μm}$$

If the adhesive layer containing the drug is too thin, the concentration of the main ingredient drug increases, which may cause drug crystals to precipitate, and the adhesive strength may weaken, which may lower marketability. On the other hand, if the thickness of the adhesive layer containing the drug is too thick, the concentration of the main ingredient drug may be lowered, which may reduce skin permeability and decrease drug efficacy.

The above drug adhesive layer (3) contains a mixture of an acrylic-based adhesive with a functional group and an acrylic-based adhesive without a functional group, and the ratio of an acrylic-based adhesive with a functional group : an acrylic-based adhesive without a functional group is 40:60 to 90:10, preferably 50:50 to 80:20 by solid weight. At this time, the acrylic-based adhesive with a functional group is an adhesive that has both functional groups of a carboxyl group (-COOH) and a hydroxyl group (-OH). When the acrylic-based adhesive is contained in the drug adhesive layer (3) at this ratio, the skin permeability is improved while the stability is also excellent, so that both stability and permeability can be improved. In this case, skin irritation can be minimized by not using a skin penetration enhancer, and for example, the skin irritation index can be 0.0.

In terms of stability, flurbiprofen may cause stability problems such as a decrease in content and the generation of a large amount of related substances when mixed with an adhesive having a functional group among acrylic-based adhesives. On the other hand, when flurbiprofen is used with an adhesive without a functional group, it is shown to have good stability (content maintenance, less generation of related substances), but in this case, skin permeability is reduced. That is, in terms of skin permeability, the drug flurbiprofen has higher skin permeability when using an acrylic-based adhesive with a functional group than when using an acrylic-based adhesive without a functional group. Therefore, the present invention provides a mixing ratio of adhesive types that can achieve excellent skin permeability of flurbiprofen while maintaining excellent stability.

In one embodiment, the acrylic-based adhesive with a functional group contained in the drug adhesive layer (3) may be contained in an amount of about 30 to 76 weight % based on the total weight of the drug adhesive layer (3), and the acrylic-based adhesive without a functional group may be contained in an amount of about 12 to 47 weight % based on the total weight of the drug adhesive layer (3).

More specifically, the drug adhesive layer (3) may include about 5 to 40 weight % of the drug, about 30 to 76 weight % of the acrylic-based adhesive with a functional group, and about 12 to 47 weight % of the acrylic-based adhesive without a functional group, based on the total weight of the drug adhesive layer (3).

More specifically, the drug adhesive layer (3) may include about 14 to 16 weight % of the drug, about 50 to 60 weight % of the acrylic-based adhesive with a functional group, and about 25 to 35 weight % of the acrylic-based adhesive without a functional group, based on the total weight of the drug adhesive layer (3).

More specifically, the drug adhesive layer (3) may include about 18 to 20 weight % of the drug, about 35 to 45 weight % of the acrylic-based adhesive with a functional group, and about 35 to 45 weight % of the acrylic-based adhesive without a functional group, based on the total weight of the drug adhesive layer (3).

The term "acrylic-based adhesive" as used herein refers to any polyacrylate, polyacrylic polymer, acrylate polymers and acrylic polymers. Acrylic-based polymers are various types of acrylic acid or ester. It can be any one of a homopolymer, a copolymer, and a terpolymer. The acrylic-based adhesive for practicing the present invention is a polymer of one or more monomers of acrylic acid and other copolymerizable monomers. Also, acrylic-based adhesives include a copolymer of alkyl acrylate and/or methacrylate and/or copolymerizable secondary monomers. The acrylic-based adhesive with a functional group may be a copolymer with a functional monomer.

The term "acrylic-based adhesive without a functional group" as used herein means an acrylic-based adhesive having no or substantially no functional or reactive moiety in the acrylic-based adhesive. These are acrylic esters that can be copolymerized with other monomers that do not generally have functional groups such as vinyl acetate.

Acrylate monomers available include acrylic acid, methacrylic acid, butyl acrylate, butyl methacrylate, hexyl acrylate, hexyl methacrylate, 2-ethylbutyl acrylate, 2-ethylbutyl methacrylate, isooctyl acrylate, isooctyl methacrylate, 2-ethylhexyl acrylate, 2-ethylhexyl methacrylate, decyl acrylate, decyl methacrylate, dodecyl acrylate, dodecyl methacrylate, tridecyl acrylate and tridecyl methacrylate.

The functional monomer that can be used and is copolymerizable with the above alkyl acrylate or methacrylate includes acrylic acid, methacrylic acid, maleic acid, maleic acid anhydride, hydroxyethyl acrylate, hydroxypropyl acrylate, acrylamide, dimethylacrylamide, acrylonitrile, dimethylaminoethyl acrylate, dimethylaminoethyl methacrylate, t-butylaminoethyl acrylate, t-butylaminoethyl methacrylate, methoxyethyl acrylates and methoxyethyl methacrylate.

As a suitable acrylic-based adhesive, an acrylic-based adhesive with a functional group may be used, such as commercially available Duro-Tak adhesive, for example, but not limited to, Duro-Tak 87-2074, Duro-Tak 87-2979, etc.

As a suitable acrylic-based adhesive, an acrylic-based adhesive without a functional group may be used, such as commercially available Unitac NF-04, Duro-Tak 87-9301, Duro-Tak 87-6911, Duro-Tak 87-6908, Duro-Tak 87-4098, Duro-Tak 87-900A, etc., but is not limited thereto.

In the present invention, the hydrophobic adhesive layer (2) has a structure in which one side is in contact with the elastic backing layer (1) and the other side is in contact with the drug adhesive layer (3) (see Fig. 1), and contains a rubber-based adhesive.

In one embodiment, the rubber-based adhesive may be one or more adhesive selected from the group consisting of a polyisobutylene adhesive, a styrene-isoprene-styrene adhesive, and a styrene-butadiene-styrene adhesive.

In one embodiment, the thickness of the hydrophobic adhesive layer (rubber adhesive layer, rubber layer) (2) is suitably about 10 to 100 $\mu$m. It may be preferably about 20 to 60 $\mu$m, about 35 to 45 $\mu$m, more preferably about 38 to 42 $\mu$m, and most preferably about 39 $\mu$m.

In one embodiment, the hydrophobic adhesive layer (2) and the drug adhesive layer (3) may be present in a ratio of hydrophobic adhesive layer (2) : drug adhesive layer (3) being 50:50 to 80:20 % by solid weight. That is, the ratio of the acrylic layer may be about 50 to 20 weight % with respect to the total weight of the hydrophobic adhesive layer (2) and the drug adhesive layer (3). Preferably, the ratio of the rubber layer : acrylic layer may be 55:45 to 70:30. That is, the ratio of the acrylic layer may be about 45 to 30 weight % with respect to the total weight of the rubber layer and the acrylic layer.

In the present invention, the elastic backing layer (1) is in contact with one side of the hydrophobic adhesive layer (2), and the other side is exposed to the outside of the transdermal delivery preparation (see Fig. 1). The elastic backing layer (1) is made of a woven or nonwoven fabric having elasticity in one or both directions, and may be one or more selected from the group consisting of polyester, polyethylene (PE), nylon, polypropylene (PP), polyethylene terephthalate (PET), ethylene vinyl acetate (EVA), nonwoven/PET composite, PET/PE composite, and PET/EVA composite.

In the present invention, the release layer (4) is in contact with one side of the drug adhesive layer (3), and the other side is exposed to the outside of the transdermal delivery preparation (see Fig. 1). The release layer (4) is a layer that protects the drug adhesive layer (3) before using the transdermal delivery preparation, and is peeled off before application to the skin. The release layer (4) may be a release layer of one or more selected from the group consisting of a polyethylene terephthalate (PET) film, a polyethylene (PE) film, a polyester film, a polyvinyl chloride (PVC) film, a polyvinylidene chloride film, a polyethylene/paper composite, a PET/PE composite, and a PET/EVA composite, surface-treated with a silicone or fluorine treatment agent.

The multilayered transdermal delivery preparation of the present invention can be prepared into the matrix structure described above by a commonly used plaster preparing method, and examples of the preparing method are described in the Examples below, but are not limited thereto.

Hereinafter, the present invention is described in more detail through Examples and Test Examples. However, the following Examples and Test Examples are intended to illustrate the present invention, and the scope of the present invention is not limited thereto.

### Examples 1 ~ 3. Preparation of plaster with drug adhesive layer

In the following Test Example 1, three types of adhesives having excellent stability and adhesive properties were selected, and by applying them, plasters having drug adhesive layers of Examples 1 to 3 of Table 1 below were prepared (see Fig. 1). As comparative examples, plasters of Comparative Examples 1 to 3 were prepared by changing the ratio of the adhesive.

As shown in Fig. 1, the plaster had a structure in which had a hydrophobic adhesive layer (2) under an elastic backing layer (1), a drug-containing adhesive layer (3) under that, and a release layer (4) under the drug adhesive layer, so that the release layer can be removed when attached to the skin.

[Table 1]

| Drug-containing adhesive layer | | Comparative Example 1 (F only) | | Example 1 (F : NF= 80:20) | | | | Example 2 (F : NF= 65:35) | | Example3 (F : NF= 50:50) | | Comparative Example 2 (F : NF= 20:80) | | Comparative Example 3 (NF only) | | | |
|---|---|---|---|---|---|---|---|---|---|---|---|---|---|---|---|---|---|
| | | | | Example 1-1 | | Example 1-2 | | | | | | | | Comparative Example 3-1 | | Comparative Example 3-2 | |
| Ingredient | | (mg/p) | weight ratio (%) | (mg/p) | weight ratio (%) | (mg/p) | weight ratio (%) | (mg/p) | weight ratio (%) | (mg/p) | weight ratio (%) | (mg/p) | weight ratio (%) | (mg/p) | weight ratio (%) | (mg/p) | weight ratio (%) |
| Drug | Flurbiprofen | 20.0 | 14.5% | 20.0 | 14.5% | 20.0 | 14.5% | 20.0 | 14.5% | 20.0 | 14.5% | 20.0 | 14.5% | 20.0 | 14.5% | 20.0 | 14.5% |
| Function al adhe-sive | Duro-Tak 87-2074 | 118.0 | 85.5% | 94.4 | 68.3% | 94.4 | 68.3% | 76.6 | 55.5% | 58.9 | 42.7% | 23.6 | 17.1% | | | | |
| Non-func-tion al ad-hesive | Duro-Tak 87-9301 | | | 23.8 | 17.2% | | | | | | | 94.6 | 68.5% | 118.0 | 85.5% | | |
| | Unitac NF04 | | | | | 23.8 | 17.2% | 41.4 | 30.0% | 59.0 | 42.8% | | | | | 118.0 | 85.5% |
| **Total** | | **138.0** | 100.0% | **138.2** | 100% | **138.2** | 100% | **138.0** | 100% | **137.9** | 100.0% | **138.1** | 100.0% | **138.0** | 100.0% | **138.0** | 100.0% |

F= Functional, acrylic-based adhesive with a functional group

NF=Non Functional, acrylic-based adhesive without a functional group (mg/p)= mg/plaster

To prepare a hydrophobic adhesive layer, the rubber adhesive was uniformly mixed, then applied to a silicone-treated PET film and dried. An elastic backing layer was placed on top and pressed.

To prepare a drug-containing adhesive layer, the drug and adhesive were uniformly mixed and then applied to a silicone-treated PET film and dried.

The release layer (silicon-treated PET film) was removed from the pre-fabricated hydrophobic adhesive layer and laminated onto the drug-containing adhesive layer to prepare the plaster products of Examples 1 to 3 and Comparative Examples 1 to 3, respectively.

**Test Example 1. Comparison of related substance generation under stress storage conditions (60°C, 80% RH) by mixing the main ingredient flurbiprofen and each acrylic-based adhesive**

When the main drug ingredient, flurbiprofen, in the drug adhesive layer comes into contact with the acrylic-based adhesive, related substances are generated. The amount of the related substances generated may vary depending on the type of functional group of the acrylic-based adhesive and the type of residual monomer.

A screening experiment was conducted to find an acrylic-based adhesive that produces less related substances (having excellent stability) when reacting with the drug flurbiprofen.

Flurbiprofen and an acrylic-based adhesive were mixed in a weight ratio of 1:1 and left under stress storage conditions (temperature 60°C, humidity 80% RH), and the related substances generated after 2, 4, and 6 weeks were analyzed.

The related substances analysis equipment and conditions are as follows.

- Analytical equipment: Ultra-high performance liquid chromatography (UPLC)
- Mobile phase: pH 2.5 buffer : acetonitrile = 7:3 (v/v)
- Detector: UV wavelength 254 nm

The total amount of related substances (%) is shown in Table 2 below, and the maximum value among individual related substances (Individual Max) is shown in < >.

When applying the global standard (international standard) according to the ICH guideline, individual related substances (%), not the total related substances, are managed so that they do not exceed 0.5% or 1.0%. Therefore, it is important to check the maximum value of individual related substances together.

In this test example, the main ingredient flurbiprofen and each adhesive were mixed in the same ratio, so the results may be harsher than the results of related substance generation in the actual manufactured product. However, a comparison of the application of each adhesive suggests that a more stable adhesive will be expected when applied to flurbiprofen.

[Table 2]

| Functional group | Type | Adhesive type | Total amount of related substances (%) | | | |
|---|---|---|---|---|---|---|
| | | | initial | 2 weeks under stress | 4 weeks under stress | 6 weeks under stress |
| With | -COOH | Duro-Tak 87-2196 | 0.00% | 0.64% | 2.10% <1.96%> | 4.59% <4.38%> |
| | | Duro-Tak 87-2051 | 0.00% | 0.29% | 1.17% <0.96%> | 2.84% <2.32%> |
| | -OH | Duro-Tak 87-2516 | 0.00% | 0.43% <0.26%> | 1.08% <0.79%> | 2.09% <1.66%> |
| | | Zelva GMS 788 NA | 0.00% | 0.17% | 0.67% <0.57%> | 1.38% <1.27%> |
| | -OH, -COOH | Duro-Tak 87-2074 | 0.11% | 0.28% <0.15%> | 0.91% <0.38%> | 1.71% <0.67%> |
| Without | | Unitech NF-04 | 0.00% | 0.50% <0.24%> | 1.02% <0.35%> | 1.44% <0.60%> |
| | | Duro-Tak 87-9301 | 0.00% | 0.00% | 0.06% | 0.26% <0.13%> |
| | | Duro-Tak 87-6911 | 0.00% | 0.10% | 0.10% | 0.21% <0.13%> |
| | | Duro-Tak 87-6908 | 0.00% | 0.00% | 0.00% | 0.00% |

As shown in Table 2 above, it can be confirmed that there is a clear difference in stability depending on the presence or absence of a functional group. The adhesive without a functional group was the most stable when applied, and among the adhesives with functional groups, the adhesive having both a hydroxyl group (-OH) and a carboxyl group

(-COOH) was the most stable when applied. In addition, the adhesive with only a hydroxyl group (-OH) was more stable when applied than the adhesive with only a carboxyl group (-COOH). Therefore, when applying the adhesive, the stability ranking according to the functional group can be expressed in the order of non-functional group > (-OH, -COOH) > (-OH) > (-COOH).

Through adhesive screening experiments, adhesives with excellent stability were selected, and among them, three types of adhesives with excellent adhesive properties (Duro-Tak 87-2074, Duro-Tak 87-9301, and Unitech NF-04) were selected to prepare the example plaster.

**Test Example 2. Evaluation of the precipitation of Active Pharmaceutical Ingredient in the plaster**

In order to measure the difference in physical properties (whether crystals are precipitated and adhesive strength) according to the change in concentration of the Active Pharmaceutical Ingredient (API) in the acrylic-based adhesive layer containing the drug, the thickness of the hydrophobic adhesive layer was fixed at 35 to 45 μm, and the plaster was prepared by changing the thickness of the drug-containing adhesive layer.

The physical properties of the product, including whether crystals were precipitated, were checked for the prepared plaster and are shown in Table 3 below. Those where crystals were precipitated were marked with O, those where only a small amount of crystals were visible were marked with △, and those where no crystals were precipitated were marked with X.

[Table 3]

| Thickness of hydrophobic adhesive layer | API concentration within the acrylic-based adhesive layer (weight %) | Whether or not precipitated | | | Check the adhesiveness |
|---|---|---|---|---|---|
| | | cold storage | long term | accelera ted storage | |
| 35~45 μm | 7% | X | X | X | Commonly |
| | 9% | X | X | X | |
| | 11% | X | X | X | |
| | 13% | X | X | X | Excellent adhesion |
| | 15% | X | X | X | |
| | 17% | X | X | X | |
| | 19% | X | X | X | |
| | 21% | X | X | X | |
| | 22% | X | X | △ | Presence of samples with weak adhesion Adhesion weakens due to crystal precipitation |
| | 23% | X | △ | O | |
| | 25% | △ | O | O | |
| | 30% | O | O | O | |
| | 40% | O | O | O | |

As shown in Table 3 above, it was confirmed that crystals were precipitated when the concentration of the main drug ingredient in the drug adhesive layer (acrylic-based adhesive layer) was about 23 weight % or more.

No crystal precipitation occurred in all of the plasters prepared in Comparative Examples 1 to 3 and Examples 1 to 3, and it was confirmed that the adhesive strength was excellent.

**Test Example 3. Skin penetration test of products applying drug adhesive layers of Comparative Examples 1 to 3 and Examples 1 to 3**

A skin penetration test was conducted as follows for products to which a drug-containing adhesive layer was applied as in Comparative Examples 1 to 3 and Examples 1 to 3.

Franz cell (Franz type diffusion cell) device was used, and human cadaver epidermis was used as the skin. PBS (phosphate buffer, pH 7.4) was added to the receptor part below the Franz cell, and the temperature was maintained at 32°C and the PBS was stirred at 600 rpm. The skin was placed on the receptor of the Franz cell, and the prepared plaster

was placed on top of each, and then firmly fixed with a clamp so that it would not move. To quantify the drug penetrated over time, 0.25 ml of the receptor liquid was taken at regular intervals and quantified by HPLC. An equal amount of PBS taken from the receptor, 0.25 ml, was filled again to the liquid.

The equipment and conditions for analyzing drug content are as follows.

- Analytical equipment: High-performance liquid chromatography (HPLC)
- Mobile phase: pH 2.5 buffer: Acetonitrile =1:1 (v/v)
- Detector: UV wavelength 254 nm

The results of the skin penetration test are shown in Table 4 and Table 5 below. This shows the in-vitro permeability of the drug.

Table 4 below shows skin penetration amount (μg/cm$^2$) and skin absorption rate (μg/cm$^2$/ hr) of flurbiprofen in products to which Comparative Examples 1 to 3 and Examples 1 to 3 were applied.

【Table 4】

| Penetration amount / Time | Comparative Example 1 | Example 1 | | Example 2 | Example 3 | Comparative Example 2 | Comparative Example 3 | |
|---|---|---|---|---|---|---|---|---|
| | | Example 1-1 | Example 1-2 | | | | Comparative Example 3-1 | Comparative Example 3-2 |
| 0 hr | 0.0 | 0.0 | 0.0 | 0.0 | 0.0 | 0.0 | 0.0 | 0.0 |
| 4 hr | 12.0 | 22.9 | 21.1 | 20.1 | 16.0 | 6.9 | 4.5 | 4.0 |
| 8 hr | 24.3 | 46.3 | 43.1 | 40.9 | 29.1 | 17.1 | 10.1 | 9.7 |
| 12 hr | 36.3 | 64.6 | 65.1 | 60.2 | 42.8 | 24.3 | 17.1 | 13.3 |
| 24 hr | 69.4 | 109.1 | 105.7 | 97.6 | 76.9 | 53.7 | 37.8 | 31.4 |
| Skin absorption rate (μg/cm$^2$/hr) | 2.9 | 4.5 | 4.4 | 4.1 | 3.2 | 2.2 | 1.6 | 1.3 |

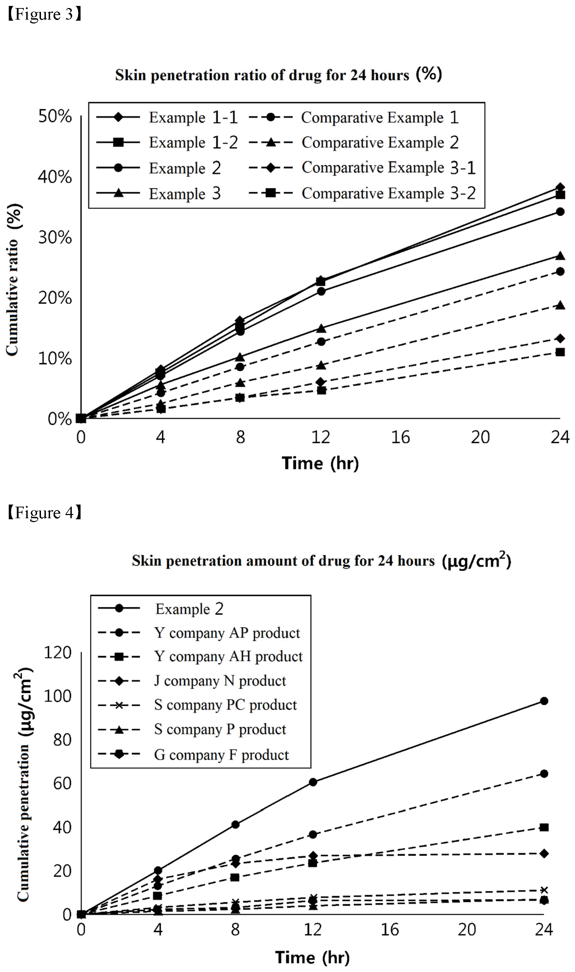

The drug content of flurbiprofen in each prepared plaster is 20mg/70cm$^2$, which is 285.714 μg/cm$^2$, and taking this as 100%, the skin penetration ratio (%) of flurbiprofen in each plaster was calculated. Table 5 below shows the skin penetration ratio (%) of flurbiprofen of products to which Comparative Examples 1 to 3 and Examples 1 to 3 were applied. In addition, In addition, the skin penetration amount (μg/cm$^2$) of flurbiprofen in each plaster is shown graphically in Fig. 2, and the skin penetration rate (%) of flurbiprofen in each plaster is shown graphically in Fig. 3.

【Table 5】

| Penetration ratio / Time | Comparative Example 1 | Example 1 | | Example 2 | Example 3 | Comparative Example 2 | Comparative Example 3 | |
|---|---|---|---|---|---|---|---|---|
| | | Example 1-1 | Example 1-2 | | | | Comparative Example 3-1 | Comparative Example 3-2 |
| 0 hr | 0.0% | 0.0% | 0.0% | 0.0% | 0.0% | 0.0% | 0.0% | 0.0% |
| 4 hr | 4.2% | 8.0% | 7.4% | 7.0% | 5.6% | 2.4% | 1.6% | 1.4% |
| 8 hr | 8.5% | 16.2% | 15.1% | 14.3% | 10.2% | 6.0% | 3.6% | 3.4% |
| 12 hr | 12.7% | 22.6% | 22.8% | 21.0% | 15.0% | 8.9% | 6.0% | 4.7% |
| 24 hr | 24.3% | 38.2% | 37.0% | 34.2% | 26.9% | 18.8% | 13.2% | 11.0% |

As shown in Table 4 and Table 5 above (see also Figs. 2 and 3), the plasters of Example 1 (F:NF=80:20), Example 2 (F:NF=65:35), and Example 3 (F:NF=50:50) showed significantly superior skin penetration amount and skin penetration rate of flurbiprofen compared to the plasters of Comparative Example 1 (F:NF=100:0), Comparative Example 2 (F:NF=20:80), and Comparative Example 3 (F:NF=0:100).

In addition, the skin permeability of the plaster to which two types of adhesives were mixed (Examples 1, 2, and 3) was superior to that of the plaster to which a single adhesive was applied such as Comparative Examples 1 (an adhesive

with a functional group) and Comparative Examples 3 (an adhesive without a functional group). However, Comparative Example 2 (F:NF=20:80) showed a lower skin permeability than that of Comparative Example 1 (F:NF=100:0).

When one type of adhesive is applied alone, the skin permeability is better in the adhesive with a functional group (Comparative Example 1) than in the adhesive without a functional group (Comparative Example 3), and when two types of adhesives are applied in combination, the skin permeability tends to increase as the ratio of the adhesive with a functional group increases.

The results of comparing the skin penetration amount ($\mu g/cm^2$) and skin absorption rate ($\mu g/cm^2/hr$) of flurbiprofen for 24 hours between the prepared plaster of Example 2 and a commercially available product are shown in Table 6 below. In addition, the skin penetration amount ($\mu g/cm^2$) of flurbiprofen in the prepared plaster of Example 2 and in commercially available products for 24 hours are shown graphically in Fig. 4.

【Table 6】

| Time \ Product | **HANDOK Example 2** | Y company AP product | Y company AH product | J company N product | S company PC product | S company P product | G company F product |
|---|---|---|---|---|---|---|---|
| Main ingredient per unit area ($\mu g/cm^2$) | **$20mg/70cm^2$ $=286\mu g/cm^2$** | $33mg/117cm^2$ $=282\mu g/cm^2$ | $20mg/70cm^2$ $=286\mu g/cm^2$ | $20mg/70cm^2$ $=286\mu g/cm^2$ | $28mg/98cm^2$ $=286\mu g/cm^2$ | $20mg/70cm^2$ $=286\mu g/cm^2$ | $20mg/70cm^2$ $=286\mu g/cm^2$ |
| 0 hr | **0.00** | 0.0 | 0.0 | 0.0 | 0.0 | 0.0 | 0.0 |
| 4 hr | **20.1** | 13.0 | 8.5 | 16.2 | 3.2 | 1.6 | 2.2 |
| 8 hr | **40.9** | 25.2 | 16.8 | 23.2 | 5.4 | 2.9 | 3.1 |
| 12 hr | **60.2** | 36.6 | 23.4 | 26.9 | 7.5 | 4.0 | 6.3 |
| 24 hr | **97.6** | 64.2 | 39.8 | 27.9 | 11.1 | 6.8 | 6.3 |
| **Skin absorption rate ($\mu g/cm^2/hr$)** | **4.1** | 2.7 | 1.7 | 1.2 | 0.5 | 0.3 | 0.3 |

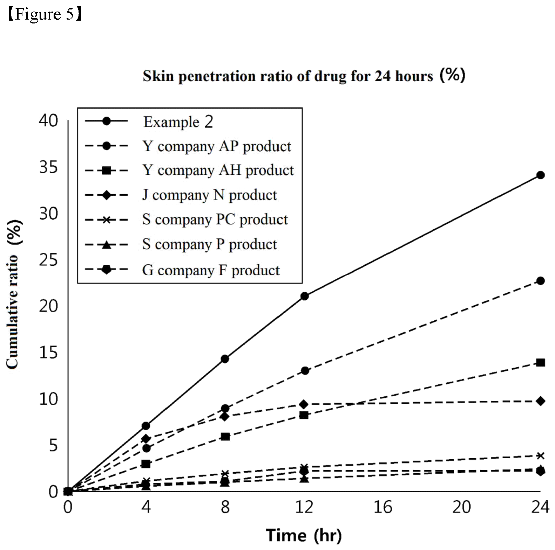

The skin penetration ratio (%) of flurbiprofen in the plaster prepared in Example 2 and in the commercially available product for 24 hours are shown in Table 7 below. In addition, the skin penetration ratio (%) of flurbiprofen in the prepared plaster of Example 2 and commercially available products for 24 hours are shown graphically in Fig. 5.

【Table 7】

| Time \ Product | **HANDOK Example 2** | Y company AP product | Y company AH product | J company N product | S company PC product | S company P product | G company F product |
|---|---|---|---|---|---|---|---|
| 0 hr | **0.0 %** | 0.0 % | 0.0 % | 0.0 % | 0.0 % | 0.0 % | 0.0 % |
| 4 hr | **7.0 %** | 4.6 % | 3.0 % | 5.7 % | 1.1 % | 0.5 % | 0.8 % |
| 8 hr | **14.3 %** | 8.9 % | 5.9 % | 8.1 % | 1.9 % | 1.0 % | 1.1 % |
| 12 hr | **21.0 %** | 13.0 % | 8.2 % | 9.4 % | 2.6 % | 1.4 % | 2.2 % |
| 24 hr | **34.1 %** | 22.8 % | 13.9 % | 9.8 % | 3.9 % | 2.4 % | 2.2 % |

As shown in Table 6 and Table 7 above (see also Figures 4 and 5), the plaster of Example 2 showed significantly superior skin penetration amount, skin absorption rate, and skin penetration ratio of flurbiprofen compared to commercially available products. In the plaster of Example 2, despite not using a penetration enhancer, it was confirmed to show better skin penetration results than commercially available products.

**Test Example 4. Preparing of plaster with changed dosage of main ingredient and skin penetration test**

Through the same process as in Test Example 2, plasters (Examples 4 and 5) were prepared with the dosage of the main ingredient flurbiprofen changed to 40 mg/70 $cm^2$ (1 sheet of plaster) according to the composition in Table 8 below.

[Table 8]

| **Drug-containing adhesive layer** | | **Example 2 (F:NF= 65:35)** | | **Example 3 (F:NF= 50:50)** | | **Example 4 (F:NF= 65:35)** | | **Example 5 (F:NF= 50:50)** | |
|---|---|---|---|---|---|---|---|---|---|
| Ingredient | | (mg/p) | Weight ratio (%) | (mg/p) | Weight ratio (%) | (mg/p) | Weight ratio (%) | (mg/p) | Weight ratio (%) |
| Drugs | Flurbiprofen | 20.0 | 14.5% | 20.0 | 14.5% | 40 | 18.8% | 40 | 18.8% |
| Functional adhesive | Duro-Tak 2074 | 76.6 | 55.5% | 58.9 | 42.7% | 112.5 | 52.8% | 86.5 | 40.6% |
| Non-functional adhesive | Unitech NF04 | 41.4 | 30.0% | 59.0 | 42.8% | 60.5 | 28.4% | 86.5 | 40.6% |
| **Total** | | **138.0** | 100.0% | **137.9** | 100.0% | **213.0** | 100.0% | **213.0** | 100.0% |

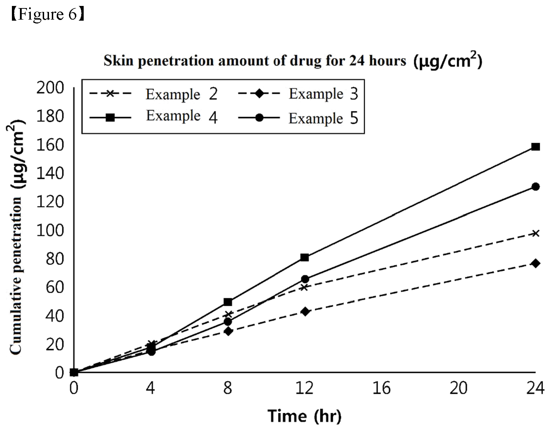

For the above prepared examples, a skin penetration test was conducted as described in Test Example 3. The skin penetration amount (μg/$cm^2$) of flurbiprofen of products to which Examples 2 to 4 were applied are shown in Table 9 below. In addition, the skin penetration ratio (%) of flurbiprofen for 24 hours of the products to which Examples 2 to 5 were applied are shown graphically in Fig. 6.

【Table 9】

| Time \ Penetration amount | Example 2 | Example 3 | Example 4 | Example 5 |
|---|---|---|---|---|
| 0 hr | 0.0 | 0.0 | **0.0** | **0.0** |
| 4 hr | 20.0 | 16.0 | **17.6** | **14.6** |
| 8 hr | 40.9 | 29.1 | **49.3** | **35.8** |
| 12 hr | 60.0 | 42.8 | **81.0** | **65.4** |
| 24 hr | 97.6 | 76.9 | **158.8** | **130.5** |
| **Skin absorption rate (μg/$cm^2$/hr)** | 4.1 | 3.2 | 6.6 | 5.4 |
| **Main ingredient per unit area (μg/$cm^2$)** | 20mg/70$cm^2$ =286μg/$cm^2$ | 20mg/70$cm^2$ =286μg/$cm^2$ | 40mg/70$cm^2$ =571μg/$cm^2$ | 40mg/70$cm^2$ =571μg/$cm^2$ |

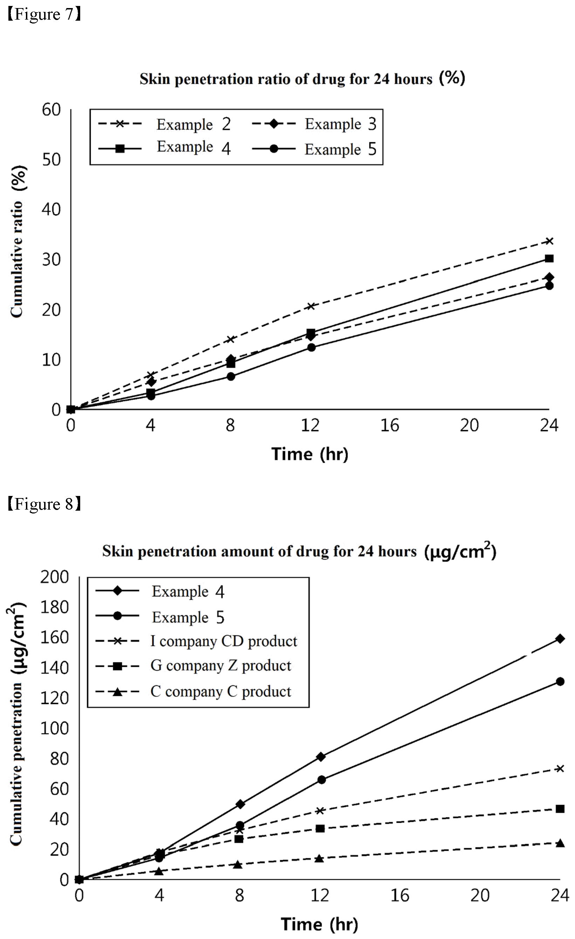

The skin penetration ratio (%) was calculated by taking the drug contents of the main ingredient flurbiprofen per unit area of 20 mg/70 $cm^2$ = 285.714 μg/$cm^2$ and 40 mg/70 $cm^2$ = 571.429 μg/$cm^2$ as 100%, respectively, and the results are shown in Table 10 below. In addition, the skin penetration ratio (%) of flurbiprofen for 24 hours of the products to which Examples 2 to 5 were applied are shown graphically in Fig. 7.

【Table 10】

| Time \ Penetration ratio | Example 2 | Example 3 | Example 4 | Example 5 |
|---|---|---|---|---|
| 0 hr | 0.0% | 0.0% | 0.0% | 0.0% |
| 4 hr | 7.0% | 5.6% | 3.4% | 2.8% |
| 8 hr | 14.0% | 10.2% | 9.5% | 6.9% |
| 12 hr | 21.0% | 15.0% | 15.6% | 12.6% |
| 24 hr | 34.2% | 26.9% | 30.7% | 25.2% |

The results of comparing skin penetration amount (μg/cm²) and skin absorption rate (μg/cm²/hr) of flurbiprofen for 24 hours between the plasters of Examples 4 and 5 and commercially available products are shown in Table 11 below. In addition, the skin penetration amount (μg/cm²) of flurbiprofen in the plasters of Examples 4 and 5 and commercially available products for 24 hours are shown graphically in Fig. 8.

[Table 11]

| Product | Example 4 | Example 5 | I company CD product | G company Z product | C company C product |
|---|---|---|---|---|---|
| Main ingredient per unit area | 40mg/ 70cm² =571μg/cm² | | 40mg/ 70cm² =571μg/cm² | 40mg/ 70cm² =571μg/cm² | 40mg/ 98cm² =408μg/cm² |
| (μg/cm²) | | | | | |
| 0 hr | 0.0 | 0.0 | 0.0 | 0.0 | 0.0 |
| 4 hr | 17.6 | 14.6 | 17.3 | 16.6 | 6.0 |
| 8 hr | 49.3 | 35.8 | 32.7 | 26.7 | 10.4 |
| 12 hr | 81.0 | 65.4 | 45.6 | 33.5 | 14.3 |
| 24 hr | 158.8 | 130.5 | 73.2 | 46.9 | 24.1 |
| Skin absorption rate (μg/cm²/hr) | 6.6 | 5.4 | 3.0 | 2.0 | 1.0 |

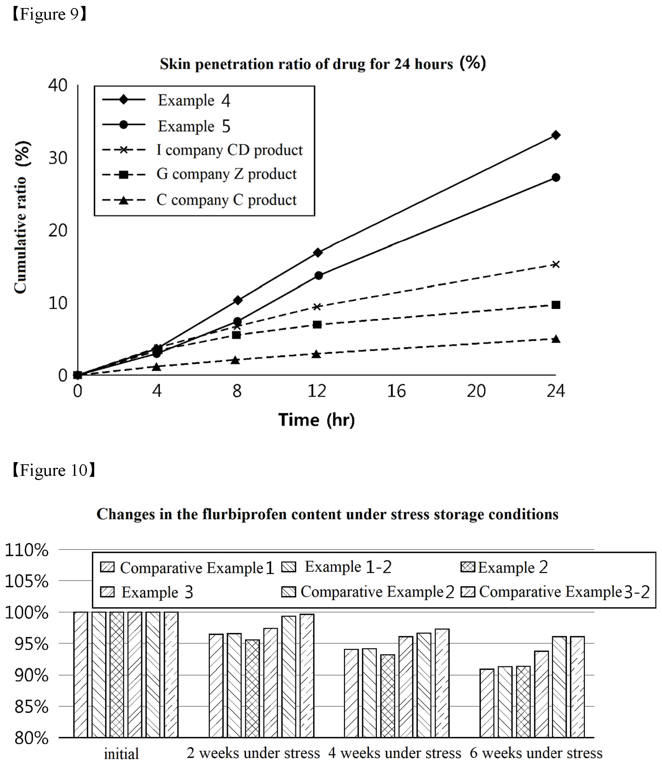

The skin penetration ratio (%) of flurbiprofen in the plasters of Examples 4 and 5 and commercially available products are shown in Table 12 below. In addition, the skin penetration rates (%) of flurbiprofen in the plasters of Examples 4 and 5 and commercially available products for 24 hours are shown graphically in Fig. 9.

[Table 12]

| Product | Example 4 | Example 5 | I company CD product | G company Z product | C company C product |
|---|---|---|---|---|---|
| Main ingredient per unit area (μg/cm²) | 40mg/ 70cm² =571μg/cm² | | 40mg/ 70cm² =571μg/cm² | 40mg/ 70cm² =571μg/cm² | 40mg/ 98cm² =408μg/cm² |
| 0 hr | 0.0% | 0.0% | 0.0% | 0.0% | 0.0% |
| 4 hr | 3.4% | 2.8% | 3.3% | 3.2% | 1.5% |
| 8 hr | 9.5% | 6.9% | 6.3% | 5.2% | 2.5% |
| 12 hr | 15.6% | 12.6% | 8.8% | 6.5% | 3.5% |
| 24 hr | 30.7% | 25.2% | 14.1% | 9.1% | 5.9% |

**Test Example 5. Content and related substance generation test of products applying drug adhesive layers of Comparative Examples 1 to 3 and Examples 1 to 4**

To evaluate the stability of products to which the drug adhesive layers of Comparative Examples 1 to 3 and Examples 1 to 4 were applied, the content and related substances changes were confirmed at initial, 2, 4, and 6 weeks under stress storage conditions (temperature 60°C, humidity 80% RH).

Table 13 below shows the change in flurbiprofen content (%) of Comparative Examples 1 to 3 and Examples 1 to 3. In addition, the change in flurbiprofen content (%) of Comparative Examples 1 to 3 and Examples 1 to 3 is shown graphically in Fig. 10.

[Table 13]

| Content (%) | initial | 2 weeks under stress | 4 weeks under stress | 6 weeks under stress |
|---|---|---|---|---|
| **Comparative Example 1** | 100.0% | 96.5% | 94.1% | 91.0% |
| **Example 1-2** | 100.0% | 96.6% | 94.2% | 91.3% |
| **Example 2** | 100.0% | 95.6% | 93.2% | 91.4% |
| **Example 3** | 100.0% | 97.5% | 96.1% | 93.8% |
| **Comparative Example 2** | 100.0% | 99.4% | 96.7% | 96.1% |
| **Comparative Example 3-2** | 100.0% | 99.7% | 97.3% | 96.1% |

At the 6-week point in the stress storage conditions test, the change in content was confirmed to be the smallest in Comparative Examples 2 and 3-2 comprising 80 to 100 weight % of the non-functional group adhesive. That is, as in Comparative Examples 2 and 3-2, the stability improved as the non-functional group ratio increased, but as confirmed in Tables 4 and 5, Comparative Examples 2 to 3 were shown to have significantly lower skin permeability than Examples 1 to 3.

It was confirmed that the content stability under stress storage conditions (temperature 60°C, humidity 80% RH) is correlated with the content stability under accelerated and long-term conditions. The predicted content results for 3 weeks under stress storage conditions have a similar correlation with the results for accelerated 6 months and long-term 24 months, so long-term data prediction is possible.

Table 14 below shows the content stability results under accelerated and long-term conditions of Example 2.

[Table 14]

| Content changes in Example 2 under stress conditions | | Content changes in Example 2 under accelerated conditions | | Content changes in Example 2 under long-term conditions | |
|---|---|---|---|---|---|
| initial | 100.0% | initial | 100.0% | initial | 100.0% |
| 2 weeks under stress | 95.6% | 2 months | 95.4% | 3 months | 99.0% |
| 4 weeks under stress | 93.2% | 4 months | 94.6% | 6 months | 98.8% |
| 6 weeks under stress | 91.4% | 6 months | 93.8% | 9 months | 98.3% |
| | | | | 12 months | 97.1% |

As a result of checking the content stability of the product prepared according to the present invention together with commercially available products from other companies, it was confirmed that the product prepared according to the present invention exhibits excellent content stability, as follows.

Table 15 below shows the change in content (%) corrected based on the initial content value of flurbiprofen of Example 2 and commercially available products under stress storage conditions as 100%.

【Table 15】

| Content / Time | HANDOK Example 2 | Y company AH product | J company N product | S company P product | G company F product | Y company AP product | S company PC product |
|---|---|---|---|---|---|---|---|
| initial | 100.0% | 100.0% | 100.0% | 100.0% | 100.0% | 100.0% | 100.0% |
| 2 weeks under stress | 95.6% | 90.5% | 93.7% | 96.1% | 95.3% | 84.0% | 91.2% |
| 4 weeks under stress | 93.2% | 86.3% | 91.9% | 92.0% | 92.1% | 83.2% | 88.6% |
| 6 weeks under stress | 91.4% | 79.1% | 87.4% | 91.4% | 84.4% | 77.9% | 78.4% |

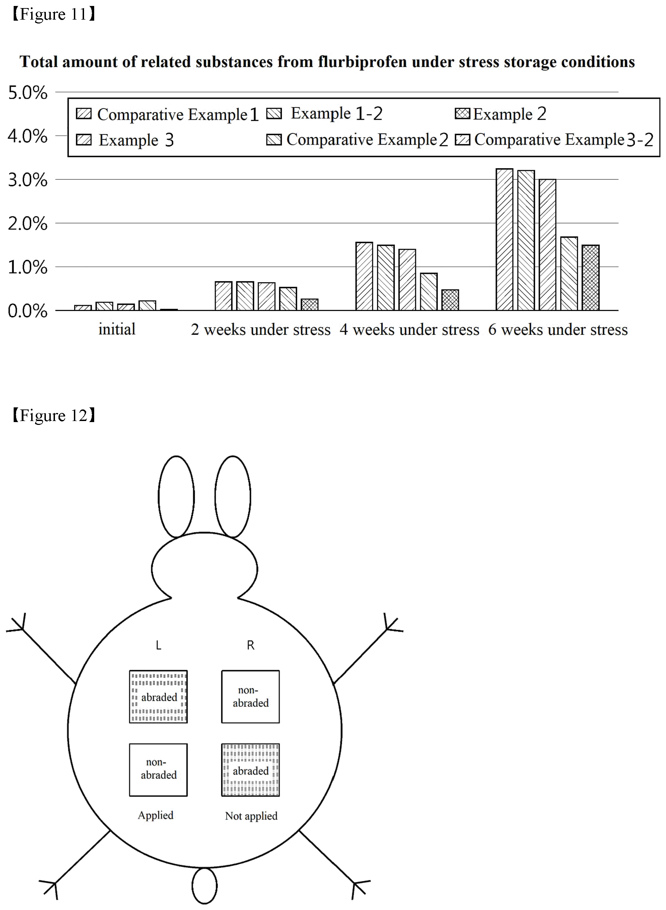

The change in the total related substances (%) of flurbiprofen in Comparative Examples 1 and 2 and Examples 1 and 2 is shown in Table 16 below. In addition, the change in the total related substances (%) of flurbiprofen in Comparative Examples 1 and 2 and Examples 1 and 2 is shown graphically in Fig. 11.

[Table 16]

| Total related substances (%) | initial | 2 weeks under stress | 4 weeks under stress | 6 weeks under stress |
|---|---|---|---|---|
| **Comparative Example 1** | 0.12% | 0.66% | 1.57% | 3.24% |
| **Example 1-1** | 0.19% | 0.66% | 1.50% | 3.21% |
| **Example 1-2** | 0.15% | 0.64% | 1.40% | 3.00% |
| **Example 2** | 0.22% | 0.53% | 0.86% | 1.69% |
| **Comparative Example 2** | 0.02% | 0.27% | 0.48% | 1.50% |

In Example 2 and Comparative Example 2 comprising 35 weight % or more of a non-functional adhesive, it was confirmed that less related substances were generated (more stable).

In addition, the results of the related substance generation under accelerated and long-term conditions of Example 2 are as shown in Table 17 below.

[Table 17]

| Generation of related substance of Example 2 under stress condition | | Generation of related substance of Example 2 under accelerated condition | | Generation of related substance of Example 2 under long-term condition | |
|---|---|---|---|---|---|
| initial | 0.22% | initial | 0.22% | initial | 0.22% |
| 2 weeks under stress | 0.53% | 2 months | 0.46% | 3 months | 0.23% |
| 4 weeks under stress | 0.86% | 4 months | 0.66% | 6 months | 0.24% |
| 6 weeks under stress | 1.69% | 6 months | 1.13% | 9 months | 0.37% |
| | | | | 12 months | 0.43% |

As a result of comparing the amount of related substances generated in products prepared according to the present invention with products commercially available from other companies, it was confirmed that while the products from other companies showed a pattern of rapid increase in the amount of related substances generated, the products prepared according to the present invention showed significantly lower amounts of related substances (data not shown).

**Test Example 6. Content stability test of plaster with changed dosage of main ingredient**

A content stability test was conducted for the plaster prepared with a dosage of 40 mg/cm$^2$ (1 sheet) of the main ingredient flurbiprofen, Example 5. Table 18 below shows the content stability results under accelerated and long-term conditions for Example 5.

[Table 18]

| Under stress storage conditions Example 5 | | Under accelerated conditions Example 5 | | Under long term conditions Example 5 | |
|---|---|---|---|---|---|
| initial | 100.0% | initial | 100.0% | initial | 100.0% |
| 2 weeks under stress | 98.1% | Acceleration 2 months | 98.3% | Long term 3 months | 99.1% |
| 4 weeks under stress | 95.4% | Acceleration 4 months | 98.0% | Long term 6 months | 98.1% |
| 6 weeks under stress | 92.4% | Acceleration 6 months | 95.4% | Long term 9 months | 97.6% |

As a result of checking the content stability of the product of Example 5 prepared according to the present invention together with commercially available products of other companies, it was confirmed that the product of Example 5 prepared according to the present invention exhibited superior content stability compared to commercially available products, as follows.

Table 19 below shows the change in content (%) corrected based on the initial content value of flurbiprofen of Example 5 and commercially available products under stress storage conditions as 100%.

[Table 19]

| Product | Example 5 | G company Z product | I company CD products | C company C Product |
|---|---|---|---|---|
| **Main ingredient per unit area ($\mu$g/cm$^2$)** | 40mg/ 70cm$^2$ =571$\mu$g/cm$^2$ | 40mg/ 70cm$^2$ =571$\mu$g/cm$^2$ | 40mg/ 70cm$^2$ =571$\mu$g/cm$^2$ | 40mg/ 98cm$^2$ =408$\mu$g/cm$^2$ |
| **initial** | 100.0% | 100.0% | 100.0% | 100.0% |
| **2 weeks under stress** | 98.1% | 94.2% | 93.1% | 94.0% |
| **4 weeks under stress** | 95.4% | 90.3% | 80.6% | 89.4% |
| **6 weeks under stress** | 92.4% | 88.6% | 72.5% | 86.0% |

**Test Example 7. Skin Irritation Test**

As a result of conducting a skin irritation test on a product prepared according to the present invention, it was confirmed to be a non-irritating product.

The skin irritation test was conducted using New Zealand White specific pathogen free (SPF) rabbits as the test system. Figure 12 shows the design for applying test materials to rabbit abdomen for skin irritation testing.

First, the rabbit's abdominal skin was shaved 24 hours before application of the test materials. As shown in Fig. 12, the left/right and upper/lower sections were divided, and the abraded skin or non-abraded skin was distributed diagonally from each other. The abraded skin was made by making an abrasion that did not bleed so that the epidermis was damaged but the dermis was not damaged.

The test material was applied directly to the skin over an area of 2.5 cm × 2.5 cm on the application area according to the Draize method. The test material was wrapped with a non-penetrating, low-irritation medical tape. It was maintained for 24 hours after a single application.

The test group included animals that were judged to be healthy during the acclimatization period and had no skin abnormalities when shaved. Their body weights were measured, and the required number of animals close to the average body weight were selected. During the test period, general symptoms were observed for all animals at least once a day, and the day of application of the test material was set as Day 0. Body weights were measured on Day 0 and Day 3.

The patch was removed at about 24 hours after application of the test material, and the contact area to which the test material had been applied was gently washed with sterile water for injection to ensure that no test material remained.

Changes such as erythema, edema, and scab formation at the application site were visually observed approximately 24 hours (30 minutes after removal of the test material) and 72 hours after application of the test material. The appearance of erythema, scab formation and edema was judged as evidence of an inflammatory reaction, with erythema being judged visually and edema being judged by light palpation. The degree of skin reaction was scored and recorded according to the skin reaction criteria at approximately 24 and 72 hours after application.

The degree of erythema and scab formation (Table 20) (1) and edema formation (Table 21) (2) were used as the evaluation criteria for skin reactions, and the presence or absence of skin reactions was determined by calculating the scores.

[Table 20]

| **Erythema and scab formation** | degree |
|---|---|
| No erythema at all | 0 |
| Very mild erythema (barely visible to the naked eye) | 1 |
| Clear erythema | 2 |
| Slightly severe erythema | 3 |
| Severe erythema (beet-colored redness) to mild scab formation (deep damage) | 4 |

[Table 21]

| **Edema formation** | degree |
|---|---|
| No edema at all | 0 |
| Very mild edema (barely visible to the naked eye) | 1 |
| Mild edema (when the swelling is distinct and the margins are clearly distinguishable) | 2 |
| Moderate edema (swelling of about 1 mm) | 3 |
| Severe edema (swelling greater than 1 mm and extending beyond the exposed area) | 4 |

The values for evaluating individual skin reactions are added up to calculate an average, and the sum of the averages divided by 4 is the Primary Irritation Index (PII). If the primary irritation index (PII) was 0.0 to 0.5, it was judged as non-irritating, 0.6 to 2.0 as mildly irritating, 2.1 to 5.0 as moderately irritating, and 5.1 to 8.0 as strongly irritating. Irritation was evaluated by considering not only the Primary Irritation Index but also general symptoms observed during the test period. A skin irritation test was conducted on two products, Example 2 and Comparative Example 2.

**(1) Results of skin irritation test in Example 2**

No general symptoms or abnormal changes in body weight related to the application of the test material were observed during the entire test period.

As a result of the skin reaction evaluation, the Primary Irritation Index (PII) was 0.

Based on the above results, the test material, flurbiprofen plaster 20 mg, was evaluated as a non-irritating product in a skin irritation test using New Zealand white rabbits under the test conditions.

**(2) Results of skin irritation test in Comparative Example 2**

Erythema and edema were observed at the site of application of the test material in all animals.

No change in body weight due to the test material was observed.

As a result of the skin reaction evaluation, the Primary Irritation Index (PII) was 2.8.

Based on the above results, the test material, flurbiprofen plaster 20 mg, was evaluated as a moderately irritating product in a skin irritation test using New Zealand white rabbits under the test conditions.

The product prepared according to the present invention (Example 2) showed the lowest value of 0.0 in the skin irritation test. Accordingly, through the present invention, a flurbiprofen plaster product without skin irritation could be derived.

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each component described as a single component may be implemented in a distributed manner, and likewise, components described as distributed may be implemented in a combined form.

The scope of the present invention is indicated by the claims set forth below, and all changes or modifications

derived from the meaning and scope of the claims and their equivalent concepts should be interpreted as being included in the scope of the present invention.

## Claims

1. A transdermal delivery preparation which is a multilayered transdermal delivery formulation, said preparation comprising:

   an elastic backing layer (1);
   a hydrophobic adhesive layer (2), the first side of the hydrophobic adhesive layer is in contact with one side of the elastic backing layer (1) and the hydrophobic adhesive layer (2) contains a rubber-based adhesive;
   a drug adhesive layer (3), the first side of the drug adhesive layer (3) is in contact with the second side of the hydrophobic adhesive layer (2), and the drug adhesive layer (3) contains an acrylic-based adhesive and a drug ; and
   a release layer (4), one side of the release layer (4) is in contact with the second side of the drug adhesive layer (3),
   wherein the acrylic-based adhesive is a mixture of an acrylic-based adhesive with a functional group and an acrylic-based adhesive without a functional group in a ratio of 40:60 to 90:10 % by solid weight,
   wherein the acrylic-based adhesive with a functional group has functional groups of carboxyl group (-COOH) and hydroxyl group (-OH),
   wherein the drug contained in the drug adhesive layer (3) is flurbiprofen.

2. The transdermal delivery preparation of Claim 1, wherein the drug contained in the drug adhesive layer (3) is present in an amount of 5 to 40 weight % based on the total weight of the drug adhesive layer (3).

3. The transdermal delivery preparation of Claim 1, wherein the acrylic-based adhesive with a functional group contained in the drug adhesive layer (3) is present in an amount of 30 to 76 weight % based on the total weight of the drug adhesive layer (3), and the acrylic-based adhesive without a functional group is present in an amount of 12 to 47 weight % based on the total weight of the drug adhesive layer (3).

4. The transdermal delivery preparation of Claim 1, wherein the rubber-based adhesive is one or more adhesive selected from the group consisting of a polyisobutylene adhesive, a styrene-isoprene-styrene adhesive, and a styrene-butadiene-styrene adhesive.

5. The transdermal delivery preparation of Claim 1, wherein hydrophobic adhesive layer (2) has a thickness of 10 to 100 μm.

6. The transdermal delivery preparation of Claim 1, wherein the hydrophobic adhesive layer (2) and the drug adhesive layer (3) are present in a ratio of hydrophobic adhesive layer (2) : drug adhesive layer (3) being 50:50 to 80:20 % by solid weight.

7. The transdermal delivery preparation of Claim 1, wherein the drug adhesive layer (3) contains 5 to 40 weight % of the drug, 30 to 76 weight % of an acrylic-based adhesive with a functional group, and 12 to 47 weight % of an acrylic-based adhesive without a functional group, based on the total weight of the drug adhesive layer (3).

8. The transdermal delivery preparation of Claim 1, wherein the drug adhesive layer (3) contains 14 to 16 weight % of the drug, 50 to 60 weight % of an acrylic-based adhesive with a functional group, and 25 to 35 weight % of an acrylic-based adhesive without a functional group, based on the total weight of the drug adhesive layer (3).

9. The transdermal delivery preparation of Claim 1, wherein the drug adhesive layer (3) contains 18 to 20 weight % of the drug, 35 to 45 weight % of an acrylic-based adhesive with a functional group, and 35 to 45 weight % of an acrylic-based adhesive without a functional group, based on the total weight of the drug adhesive layer (3).

【Figure 1】

1) an elastic backing layer
2) a hydrophobic adhesive layer
3) a drug adhesive layer
4) a release layer

【Figure 2】

Skin penetration amount of drug for 24 hours (μg/cm2)
Example 1-1
Example 1-2
Example 2
Example 3
Comparative Example 1
Comparative Example 2
Comparative Example 3-1
Comparative Example 3-2
Cumulative penetration (μg/cm2)
140
120
100
80
60
40
20
0
0
4
8
12
16
20
24
Time (hr)

【Figure 3】

Skin penetration ratio of drug for 24 hours (%)
Example 1-1
Example 1-2
Example 2
Example 3
Comparative Example 1
Comparative Example 2
Comparative Example 3-1
Comparative Example 3-2
Cumulative ratio (%)
50%
40%
30%
20%
10%
0%
0
4
8
12
16
20
24
Time (hr)

【Figure 4】

Skin penetration amount of drug for 24 hours (μg/cm²)
Example 2
Y company AP product
Y company AH product
J company N product
S company PC product
S company P product
G company F product
Cumulative penetration (μg/cm²)
120
100
80
60
40
20
0
0
4
8
12
16
20
24
Time (hr)

【Figure 5】

Skin penetration ratio of drug for 24 hours (%)
Example 2
Y company AP product
Y company AH product
J company N product
S company PC product
S company P product
G company F product
Cumulative ratio (%)
Time (hr)
0
5
10
15
20
25
30
35
40
0
4
8
12
16
20
24

【Figure 6】

Skin penetration amount of drug for 24 hours (μg/cm2)
Example 2
Example 3
Example 4
Example 5
Cumulative penetration (μg/cm2)
200
180
160
140
120
100
80
60
40
20
0
0
4
8
12
16
20
24
Time (hr)

【Figure 7】

Skin penetration ratio of drug for 24 hours (%)
Example 2
Example 3
Example 4
Example 5
Cumulative ratio (%)
60
50
40
30
20
10
0
0
4
8
12
16
20
24
Time (hr)

【Figure 8】

Skin penetration amount of drug for 24 hours (µg/cm2)
Example 4
Example 5
I company CD product
G company Z product
C company C product
Cumulative penetration (µg/cm2)
200
180
160
140
120
100
80
60
40
20
0
0
4
8
12
16
20
24
Time (hr)

【Figure 9】

Skin penetration ratio of drug for 24 hours (%)
Example 4
Example 5
I company CD product
G company Z product
C company C product
Cumulative ratio (%)
Time (hr)
0
10
20
30
40
0
4
8
12
16
20
24

【Figure 10】

Changes in the flurbiprofen content under stress storage conditions
Comparative Example 1
Example 1-2
Example 2
Example 3
Comparative Example 2
Comparative Example 3-2
110%
105%
100%
95%
90%
85%
80%
initial
2 weeks under stress
4 weeks under stress
6 weeks under stress

【Figure 11】

Total amount of related substances from flurbiprofen under stress storage conditions
5.0%
4.0%
3.0%
2.0%
1.0%
0.0%
Comparative Example 1
Example 1-2
Example 2
Example 3
Comparative Example 2
Comparative Example 3-2
initial
2 weeks under stress
4 weeks under stress
6 weeks under stress

【Figure 12】

L
R
abraded
non-abraded
non-abraded
abraded
Applied
Not applied

**INTERNATIONAL SEARCH REPORT**

International application No. **PCT/KR2024/096568**

**A. CLASSIFICATION OF SUBJECT MATTER**

**A61K 9/70**(2006.01)i; **A61K 31/192**(2006.01)i; **A61P 29/00**(2006.01)i

According to International Patent Classification (IPC) or to both national classification and IPC

**B. FIELDS SEARCHED**

Minimum documentation searched (classification system followed by classification symbols)

A61K 9/70(2006.01); A61F 13/00(2006.01); A61F 13/02(2006.01); A61K 31/445(2006.01); A61K 47/14(2006.01); A61P 29/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 플루르비프로펜(flurbiprofen), 아크릴 점착제(acryl adhesive), 관능기(functional group), 카르복실기(carboxyl group), 수산기(hydroxyl group), 경피 투여제(transdermal)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2008-0006960 A (AMOREPACIFIC CORPORATION et al.) 17 January 2008 (2008-01-17) See claims 1-4, 6-7 and 11; and figure 1. | 1-9 |
| Y | KR 10-2006-0114338 A (NOVEN PHARMACEUTICALS INC.) 06 November 2006 (2006-11-06) See claims 1, 9 and 12-14. | 1-9 |
| A | KR 10-0511492 B1 (AMOREPACIFIC CORPORATION) 31 August 2005 (2005-08-31) See entire document. | 1-9 |
| A | US 8187628 B2 (HOUZE, David et al.) 29 May 2012 (2012-05-29) See entire document. | 1-9 |
| A | KR 10-2019-0048320 A (SINSIN PHARM CO., LTD.) 09 May 2019 (2019-05-09) See entire document. | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2025** | **20 February 2025** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/096568**

**C. DOCUMENTS CONSIDERED TO BE RELEVANT**

| Category* | Citation of document, with indication, where appropriate, of the relevant passages | Relevant to claim No. |
|---|---|---|
| PX | KR 10-2722075 B1 (HANDOK INC.) 25 October 2024 (2024-10-25)<br>See claims 1-2 and 5-9.<br>(※ This document is the published patent of a priority application of the present international application.) | 1-9 |

Form PCT/ISA/210 (second sheet) (July 2022)

**INTERNATIONAL SEARCH REPORT**
**Information on patent family members**

International application No.
**PCT/KR2024/096568**

| Patent document cited in search report | | | Publication date (day/month/year) | Patent family member(s) | | | Publication date (day/month/year) |
|---|---|---|---|---|---|---|---|
| KR | 10-2008-0006960 | A | 17 January 2008 | WO | 2008-007926 | A1 | 17 January 2008 |
| KR | 10-2006-0114338 | A | 06 November 2006 | CN | 100457064 | C | 04 February 2009 |
| | | | | CN | 1901861 | A | 24 January 2007 |
| | | | | EP | 1682061 | A2 | 26 July 2006 |
| | | | | EP | 1682061 | A4 | 07 December 2011 |
| | | | | EP | 1682061 | B1 | 15 March 2017 |
| | | | | JP | 2007-509951 | A | 19 April 2007 |
| | | | | JP | 2013-237691 | A | 28 November 2013 |
| | | | | US | 2005-0169977 | A1 | 04 August 2005 |
| | | | | US | 2010-0310610 | A1 | 09 December 2010 |
| | | | | US | 2014-0243764 | A1 | 28 August 2014 |
| | | | | US | 8703175 | B2 | 22 April 2014 |
| | | | | WO | 2005-042055 | A2 | 12 May 2005 |
| | | | | WO | 2005-042055 | A3 | 25 August 2005 |
| KR | 10-0511492 | B1 | 31 August 2005 | CN | 100469367 | C | 18 March 2009 |
| | | | | JP | 2006-503877 | A | 02 February 2006 |
| | | | | KR | 10-2004-0033082 | A | 21 April 2004 |
| | | | | WO | 2004-032927 | A1 | 22 April 2004 |
| US | 8187628 | B2 | 29 May 2012 | EP | 1061900 | A1 | 27 December 2000 |
| | | | | EP | 1061900 | B1 | 02 February 2005 |
| | | | | EP | 1061900 | B2 | 09 July 2008 |
| | | | | US | 2002-0058068 | A1 | 16 May 2002 |
| | | | | US | 2003-0170195 | A1 | 11 September 2003 |
| | | | | US | 2006-0233870 | A1 | 19 October 2006 |
| | | | | US | 2006-0240087 | A1 | 26 October 2006 |
| | | | | US | 8216606 | B2 | 10 July 2012 |
| | | | | WO | 00-41538 | A2 | 20 July 2000 |
| | | | | WO | 00-41538 | A3 | 11 January 2001 |
| KR | 10-2019-0048320 | A | 09 May 2019 | None | | | |
| KR | 10-2722075 | B1 | 25 October 2024 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020230158673
- KR 1000663163